Europäisches Patentamt

European Patent Office

Office européen des brevets

(19)

(11) Publication number: **0 154 063**

A1

(12) # EUROPEAN PATENT APPLICATION

(21) Application number: 84301345.9

(22) Date of filing: 01.03.84

(51) Int. Cl.⁴: **C 07 C 1/04**
**B 01 J 29/06**

(43) Date of publication of application:
11.09.85 Bulletin 85/37

(84) Designated Contracting States:
DE FR GB IT NL

(71) Applicant: THE STANDARD OIL COMPANY
Midland Building
Cleveland, Ohio 44115(US)

(72) Inventor: Desmond, Michael Joseph
2316 Westminster Road
Cleveland Heights Ohio 44118(US)

(72) Inventor: Pepera, Marc Anthony
7304 Honeydale Drive
Northfield Center Ohio 44067(US)

(74) Representative: Smith, Sydney et al,
Elkington and Fife High Holborn House 52/54 High
Holborn
London WC1V 6SH(GB)

(54) Modified silicalite catalysts and their preparation and process for the use thereof.

(57) Catalysts comprising a silica molecular sieve modified by a metal which forms acidic oxides selected from the group consisting of Group VIB and VIIB elements and, a Fischer-Tropsch catalyst wherein the ratio of zeolite to Fischer-Tropsch component is from 0.1 to 10:1 can be employed to produce gasoline quality liquid hydrocarbons from synthesis gas. A process is employed which includes the step of contacting synthesis gas having a ratio of hydrogen to carbon oxides of from 0.1 to 10.0:1 over the foregoing catalyst at a reaction temperature of from 100°C to 500°C and at a pressure of from one atmosphere (0.1 MPa) to 200 atmospheres (20 MPa) and at a gas hourly space velocity of from 10 to 100,000. A method is also provided for the preparation of the combination catalyst. The liquid hydrocarbon product is highly aromatic, essentially free of external olefins and totally free of detectable oxygenates.

EP 0 154 063 A1

**0154063**

# MODIFIED SILICALITE CATALYSTS AND THEIR
# PREPARATION AND PROCESS FOR THE USE THEREOF

The present invention is directed toward modified silicalite catalysts and the use thereof for the production of fuel grade liquid hydrocarbons from synthesis gas. The catalysts and process disclosed are particularly suited for the production of isoparaffins, internal olefins and aromatics. A method for the synthesis of the catalyst is also provided.

Silicalite has the same structure as the alumina containing zeolite ZSM-5. The acid form of ZSM-5 is often used to convert low molecular weight alcohols or olefins to gasoline. ZSM-5 can also be used in a combination catalyst with a Fischer-Tropsch component, to give high quantities of isoparaffins and aromatics. However, silicalite does not have the acidity of ZSM-5 and its ability to "reform" Fischer-Tropsch products to isoparaffins and aromatics is greatly reduced.

The conversion of synthesis gas via Fischer-Tropsch catalysts is a well known process. Such catalysts generally produce liquid products which include components such as organic acids, aldehydes, alcohols and olefins which are undesirable for use as fuels. It is also known to employ zeolite catalysts for the conversion of various organic compounds. For example, U.S. Pats. No. 4,276,438 and 4,278,827 disclose processes for the conversion of aromatic compounds to dialkylbenzene compounds. Each process requires a particular type of zeolite catalyst which has been modified with either a Group IB or a Group IVB metal.

U.S. Pat. No. 3,775,501 discloses a process for the production of aromatics from hydrocarbons and air over a zeolite catalyst. U.S. Pat. No. 3,271,418 discloses a process for cracking petroleum gas oil to produce high octane gasoline over a zeolite catalyst where the original alkali metal has been replaced by other cations.

Others have disclosed the use of Fischer-Tropsch catalysts with zeolites in organic synthesis. U.S. Pat. No. 4,279,830, for instance, discloses a two reactor process for converting synthesis gas to oxygenates and hydrocarbons by reducing CO in the first reactor over a Fischer-Tropsch catalyst, where a zeolite is optionally present, followed by treatment in the second reactor over a zeolite catalyst.

U.S. Pat. No. 4,269,783 discloses a process for the conversion of synthesis gases to hydrocarbon mixtures such as olefinic naphtha with an aromatic content of less than 20 weight percent. The olefin plus aromatic content of such products comprises at least 50 weight percent. The zeolite component of the catalyst combination comprises not more than 0.5 weight percent alkali metal.

Lastly, U.S. Pat. No. 4,086,262 discloses a method for the conversion of synthesis gas to hydrocarbon mixtures rich in aromatics. A heterogeneous catalyst comprising zeolites and Fischer-Tropsch synthesis catalysts is employed.

Thus, while the art provides catalysts and processes for the conversion of synthesis gases and other organic feedstocks to various hydrocarbons over mixtures of zeolite and Fischer-Tropsch catalysts, it has not disclosed the preparation and use of a modified silicalite, Fischer-Tropsch combination catalyst and the use thereof to produce liquid hydrocarbons.

## DISCLOSURE OF INVENTION

It is therefore an object of the present invention to provide a process for producing gasoline quality liquid hydrocarbons from synthesis gas.

It is another object of the present invention to provide a novel catalyst for converting synthesis gas to gasoline quality liquid hydrocarbons.

It is still another object of the present invention to provide a method for the preparation of a combination catalyst for the conversion of synthesis gas to liquid hydrocarbons.

These and other objects, together with the advantages thereof over the prior art, which shall become apparent from the specification which follows, are accomplished by our invention as hereinafter described and claimed.

The catalyst of the present invention comprises a silica molecular sieve modified by a metal which forms acidic oxides selected from the group consisting of Group VIB and VIIB elements and, a Fischer-Tropsch catalyst wherein the ratio of zeolite to Fischer-Tropsch component is from about 0.1 to 10:1.

The process of the present invention produces gasoline quality liquid hydrocarbons from synthesis gas and includes the step of contacting synthesis gas having a ratio of hydrogen to carbon oxides of from about 0.1 to 10.0:1 over a combination catalyst at a reaction temperature of from about 100° C to 500° C and at a pressure of from about one atmosphere (0.1 MPa) to about 200 atmospheres (20 MPa) and at a gas hourly space velocity of from about 10 to 100,000. The combination catalyst itself comprises a silica molecular sieve modified by a metal of a Group VIB or VIIB element and, a Fischer-Tropsch catalyst wherein the ratio of zeolite to Fischer-Tropsch component is from about 0.1 to 10:1.

A method is also provided for the preparation of a combination catalyst for the conversion of synthesis gas to gasoline quality liquid hydrocarbons. It includes the steps of modifying a silica molecular sieve with a metal which forms acidic oxides selected from the group consisting of Group VIB and VIIB elements and combining the modified zeolite with a Fischer-Tropsch catalyst in a ratio of from about 0.1 to 10:1.

The method can include as further steps impregnating the silica molecular sieve with a solution of the metal, evaporating the solvent, calcining the modified silica molecular sieve at temperature ranging from about 200° to 800° C for about one to 24 hours and reducing the combination catalyst with $H_2$ and/or CO at a temperature of from about 100° C to 600° C, at $H_2$ and/or CO partial pressures ranging from 0.001 MPa to about 5.07 MPa and for a period of time of from about one to 100 hours prior to employing the catalyst for the conversion of synthesis gas.

## PREFERRED MODE FOR CARRYING OUT THE INVENTION

The catalyst of the present invention comprises a mixture or combination of a Fischer-Tropsch catalyst and a silica molecular sieve, particularly silicalite, which has been modified with a metal which forms acidic oxides selected from Groups VIB and VIIB of the Periodic Table.

Silicalite is described in U.S. Pat. No. 4,061,724 and is a nearly aluminum free zeolite of the ZSM-5 structure, the latter being a well-known medium port zeolite. The silica to aluminum molar ratio in these silica molecular sieves is on the order of more than 500:1 Si:Al ($SiO_2:Al_2O_3 \geq 1000:1$). Silicalite (II) is also suitable, it being the silica analog of zeolite ZSM-11, also a medium port zeolite. Silicalite (II) has been described by Bibby, Milestone, Aldridge, _Nature_, 280, 664 (1979). Other silica polymorphs or dealuminated zeolites are also suitable as is F-silicalite, the fluorine-containing species described in U.S. Pat. No. 4,073,865. The subject matter of the publication and two patents is incorporated herein by reference.

The silicalites are modified by impregnation with solutions, preferably aqueous or organic solutions, of Group VIB or VIIB metal salts, preferably Group VIB and particularly chromium. The metal introduced into the silicalite can range from about 0.01 to about 20 weight percent and is preferably between about 0.1 to 10 weight percent. The silicate is precalcined at temperatures ranging between about 100° C to 800° C for at least one hour to remove volatiles before being impregnated with the metal salt solution.

The solvent is subsequently evaporated to produce the impregnated, or modified, solid silicalite by a conventional method such as roto-evaporation. The metal impregnated solid is then dried to remove trapped solvents and then calcined at temperatures between 300° and 800° C and preferably from 400° to 700° C. Calcining times range from about one to 24 hours. The calcined metal containing silicalite is then combined with a supported or unsupported Fischer-Tropsch catalyst, which includes any such catalyst known in the art, either as an intimate mixture or as separate particles. The Fischer-Tropsch catalysts include all of those generally known in the art. The catalysts employed in the examples were prepared according to the methods outlined in "The Fischer Tropsch and Related Syntheses" by H. H. Storch, N. Golumbic and R. B. Anderson, (John Wiley and Sons, Inc., 1951) the contents of which are incorporated herein by reference.

The ratio of modified sieve to Fischer-Tropsch component may be from about 0.1 to 10 and preferably between about 0.5 to 5:1 by weight. The mixture of Fischer-Tropsch component and modified sieve can be intimate or present as separate particles in the reactor. When the two components are combined as an intimate mixture, the powders of the components (greater than 30 mesh) are combined and thoroughly mixed in the desired ratio and a lubricant such as graphite can be added to the mix. One to two weight percent based upon the total catalyst weight is sufficient. The mixture is then pressed into pellets or wetted and extruded, followed by drying and calcination. Pelleted catalysts are broken into 10-30 mesh particles before placement into the reactor.

The catalyst is finally placed in the reactor and the Fischer-Tropsch component activated in a known manner such as by reduction with hydrogen, carbon monoxide or a combination with or without the presence of a diluent such as nitrogen. Reduction conditions are chosen to optimize the activity of the particular Fischer-Tropsch component present in the catalyst. They normally include heating to a temperature of at least 100° C up to about 600° C at $H_2$ and/or CO partial pressures ranging from 0.001 MPa to about 5.07 MPa and for a period of time of from about one to 100 hours.

In the process of the present invention, synthesis gas, _i.e._, a mixture of hydrogen and carbon monoxide, in a ratio of 0.1 to 10:1 $H_2$/CO and preferably between 0.25 to 2:1 $H_2$/CO, is reacted in the presence of the novel catalyst in the vapor phase. The conversion can be conducted in either fluid bed or fixed bed reactors, the latter being exemplified herein, and the process is applicable to continuous or batch type operation.

The reaction temperature should be maintained between temperatures from about 100° to 500° C, preferably from about 150° to 350° C. Pressures may be from ambient to about 200 atmospheres (20 MPa), preferably from ambient to about 30 atmospheres (3 MPa). Synthesis gas space velocities can range from about ten to 100,000 GHSV, or volumes of gas passed over one equivalent volume of catalyst per hour, and preferably from about 100 to 10,000 GHSV.

The use of a catalyst containing Cr-Silicalite (about 3 percent Cr) with an Fe based Fischer-Tropsch catalyst results in a liquid organic product containing large amounts of isoparaffins and aromatics. This result is much superior to the reforming ability of silicalite in an unpromoted state and is also better in producing aromatics than a combination catalyst containing HZSM-5 ($SiO_2/Al_2O_3 \sim 70$).

In the examples reported hereinbelow, a catalyst of the present invention was prepared in a two step process as follows:

### Step I

10.8 gms of $Cr(NO_3)_3 \cdot 6H_2O$ was dissolved in 300 ml of 2:1 water-acetone. To this solution 50 gm of silicalite which had been precalcined for 3 hours at 500° C and 1 hour at 600° C was added. The mixture was placed on a roto evaporation unit and the acetone slowly removed over a period of 20 minutes. The system was heated over a water bath and the water solvent was removed over the next 90 minutes. The moist solid was dried overnight (about 17 hours) at 150° C, and then calcined for 4 hours at 600° C to decompose the Cr nitrate. The expected Cr content of the catalyst was approximately 3.0 percent. The modified silicalite was designated Cr-Silicalite.

### Step II

Equal weights of a conventional Fischer-Tropsch catalyst, 75Fe(II):25Fe(III):20Cu(II) and Cr-Silicalite were mixed·with 2 percent graphite and thoroughly mixed on a roll-mill. The powder was then pressed into pellets. The pellets were broken up and screened to give a 10-30 mesh fraction which was isolated and calcined. The catalyst formed is one of the present invention and is designated as Catalyst A.

Catalyst A was reduced and then run in a fixed bed reactor for the conversion of synthesis gas as follows: 20 cc of Catalyst A was reduced at ambient pressure at a temperature of 250° C under a flow of 50 SCCM (standard cubic centimeters) $H_2$, 50 SCCM CO and 900 SCCM $N_2$ over-night. The pressure was increased to 1.04 MPa and flows of 50 SCCM $H_2$ and 50 SCCM CO were introduced. The bath temperature was increased to 270° C and a 1159 minute run was conducted. A gas contraction of 10.7 percent was observed. The $C_5^+$ liquid yield was 0.06 gms per hour.

For purposes of comparison, two catalysts not part of the present invention, designated as B and C, were also prepared by substituting zeolite and unmodified silicalite for the Cr-Silicalite component of the invention. Preparation was as follows:

### Catalyst B

The procedure of Step II was employed with the acid form of a medium port zeolite, HZSM-5 ($SiO_2:Al_2O_3 \sim 70:1$) substituted for the Cr-Silicalite of Step I.

### Catalyst C

The procedure of Step II was employed with silicalite ($SiO_2:Al_2O_3>1000$) substituted for the Cr-Silicalite of Step I.

In similar fashion, Catalysts B and C were reduced and run in a fixed bed reactor as follows: 20 cc of Catalyst B was reduced at ambient pressure at a temperature of 250° C under a flow of 50 SCCM $H_2$, 50 SCCM CO and 900 SCCM $N_2$ overnight. The pressure was increased to 1.04 MPa and flows of 50 SCCM $H_2$ and 50 SCCM CO were introduced. The bath temperature was 240° C and a 931 minute run was conducted. The gas contraction observed was 11.5 percent and the $C_5^+$ liquid yield was 0.02 gms per hour. The bath temperature was increased to 270° C and a 1539 minute run was conducted. The gas contraction observed was 37.4 percent and the $C_5^+$ yield was 0.07 gms per hour.

20 cc of Catalyst C was reduced at ambient pressure at a temperature of 250° C under a flow of 50 SCCM $H_2$, 50 SCCM CO and 900 SCCM $N_2$ overnight. The pressure was increased to 1.04 MPa and flows of 50 SCCM $H_2$ and 50 SCCM CO were introduced. The bath temperature was 251° C and a 217 minute run was conducted. The gas contraction observed was 29.4 percent and the $C_5^+$ liquid yield was 0.17 gms per hour. The bath temperature was increased to 260° C and a 937 minute run was conducted. The gas contraction observed was 45.7 percent and 0.35 gms of $C_5^+$ product was produced per hour.

The products obtained were subjected to analysis, the results of which have been summarized in Table I. The amounts of each product reported should be multiplied by $10^{-4}$ to obtain moles/gm.

0154063

## TABLE I

### Products Comparisons for Catalysts A, B and C

| Catalyst | A | B | | C | |
|---|---|---|---|---|---|
| | Cr-Silicate + Fischer-Tropsch | HZSM-5 + Fischer-Tropsch | | Silicalite + Fischer-Tropsch | |
| Temperature | 270° C | 240° C | 270° C | 251° C | 260° C |
| Vinyl Olefin | 0.0 | 1.1 | 1.3 | 9.0 | 7.8 |
| Internal Olefin | 3.6 | 9.8 | 8.2 | 6.8 | 9.7 |
| Vinylidene Olefin | 0 | 1.5 | 2.6 | 0.8 | 1.2 |
| Trisubstituted Olefin | 2.8 | 16.1 | 20.9 | 2.0 | 3.3 |
| Alcohol | 0.0 | 0.0 | 0.0 | 25.3 | 7.8 |
| Ester | 0.0 | 0.0 | 0.0 | 1.5 | 0.8 |
| Aromatic | 22.6 | 8.0 | 15.9 | 4.0 | 2.4 |
| $CH_2/CH_3$ | 2.1 | 2.3 | 2.6 | 2.8 | 3.4 |

The products obtained from Catalyst A would be the best for fuels as they contained no oxygenates and had the lowest $CH_2/CH_3$ ratio for the three catalysts employed. The $CH_2/CH_3$ ratio can be used to determine the chain length or extent of branching. When this number is compared with boiling point fraction data (not reported in Table I), it is possible to determine branching. Generally speaking a ratio of less than 2.5 is good for gasoline fractions and 2.0 is excellent. A value of 2.1 was observed for Catalyst A.

Catalyst A was next compared further against Catalysts B and C as well as other combination catalysts not part of the present invention and three conventional Fischer-Tropsch catalyst compositions which did not contain any silicalite or zeolite. The comparison catalysts included the following: Cobalt Fischer-Tropsch catalysts alone over different supports; Iron Fischer-Tropsch catalyst alone; Cobalt Fischer-Tropsch catalyst with zeolite, 50/50 mixture (Catalyst B); Cobalt Fischer-Tropsch catalyst with silicalite, 50/50 mixture (Catalyst C); Cobalt Fischer-Tropsch catalyst with silicalite 33.3/66.7 mixture (Catalyst D); Iron Fischer-Tropsch catalyst with zeolite, 50/50 mixture (Catalyst E); Iron Fischer-Tropsch catalyst with silicalite 50/50 mixture (Catalyst F); and, Iron Fischer-Tropsch catalyst with silicalite 50/50 mixture (Catalyst G). Catalysts F and G were different in that the silicalite of Catalyst G was obtained from Union Carbide (S-115) while the silicalite of Catalyst F was prepared under the direction of the inventors named herein. More specifically, the catalysts employed were as follows:

$$\underline{\text{Examples No. 1-6}}$$
$$100Co:5ThO_2:8MgO:200Kieselguhr \text{ on alundum}$$
$$\underline{\text{Examples No. 7-8}}$$
$$100Co:5ThO_2:8MgO:200Kieselguhr(10cc)+10cc-glass \text{ beads}$$
$$\underline{\text{Examples No. 9-12}}$$
$$75Fe(II):25Fe(III):20Cu(II)$$
$$\underline{\text{Examples No. 13-15}}$$
Catalyst B:  $50\%(100Co:10MgO:5ThO_2:100Kieselguhr)/50\%ZSM-5$
$$\underline{\text{Examples No. 16-19}}$$
Catalyst C:  $50\%(100Co:10MgO:5ThO_2:100Kieselguhr)/50\%silicalite$
$$\underline{\text{Examples No. 20-22}}$$
Catalyst D:  $1(100Co:8MgO:5ThO_2:100Kieselguhr)/2 \text{ silicalite}$
$$\underline{\text{Examples No. 23-28}}$$
Catalyst E:  $50\%[75Fe(II):25Fe(III):20Cu(II)]/50\%ZSM-5$
$$\underline{\text{Examples No. 29-31}}$$
Catalyst F:  $50\%[75Fe(II):25Fe(III):20Cu(II)]/50\% \text{ silicalite}$
$$\underline{\text{Examples No. 32-34}}$$
Catalyst G:  $50\%[75Fe(II):25Fe(III):20Cu(II)]/50\% \text{ silicalite}$
$$\underline{\text{Examples No. 35-36}}$$
Catalyst A:  $50\%[75Fe(II):25Fe(III):20Cu(II)]/50\%Cr-Silicalit$

In each series of examples, 20 cc of a specific catalyst was selected, placed in the reactor after appropriate reduction of its Fischer-Tropsch component and then subjected to multiple runs with increasing time being reported in Table III.

The Fischer-Tropsch catalysts were first reduced at ambient pressure or greater under gases at temperatures and for times as set forth in Table II. The other catalysts were reduced, usually at ambient pressure under a flow of $N_2$, $H_2$ and CO, 900SCCM:50SCCM:50 SCCM, respectively for 22.5 hours at 250° C. Synthesis gas conversion reactions were subsequently conducted at a bath temperature of 252° C and 1.04 MPa pressure unless otherwise stated. Reductions of the Fischer-Tropsch components of Catalysts A-G appear in Table III for the first numbered example following each new catalyst.

TABLE II

Reduction Conditions for Fischer-Tropsch Catalysts

| Ex. No. | T °C | P MPa gauge | Gas | Time Hrs. |
|---------|------|-------------|-----|-----------|
| 1 | 350 | 1.04 | $H_2$ | 18 |
| 2 | 350 | 1.04 | $H_2$ | 18 |
| 3 | 350 | Amb. | $H_2$ | 4 |
| 4 | 350 | 1.04 | $H_2$ | 16 |
| 5 | 350 | 1.04 | $H_2$ | 16 |
| 6 | 350 | 1.04 | $H_2$ | 16 |
| 7 | 400 | Amb. | $3:1 H_2/N_2$ | 17 |
| 8 | 400 | Amb. | $3:1 H_2/N_2$ | 17 |
| 9 | 250 | 1.04 | $H_2$ | 22 |
| 10 | 250 | 1.04 | $H_2$ | 22 |
| 11 | 250 | 1.04 | $H_2$ | 22 |
| 12 | 250 | 1.04 | $H_2$ | 22 |

Reactions of synthesis gas over the catalysts were conducted under conditions set forth in Table III which reports grams of organic liquid produced per hour. Gas contraction reported in Table III is determined as follows: [1-(volume gas out/volume gas in)]X100. Feed of synthesis gas is given as SCCM (standard cubic centimeters). Analysis of the liquid products by NMR was conducted and has been reported in Table IV. The amounts present equal

$1\times10^{-4}$ moles/gm of liquid. Lastly, Table V presents per pass conversion and percent selectivity. Calculations of the percent of CO converted to a particular product (% ppc) and percent selectivity (% sel) for the gaseous products of Examples were determined as follows:

% ppc = [moles of CO in product/moles of CO fed] X 100

% sel = [SCCM product/SCCM CO (converted)] X 100

TABLE III

## Synthesis Gas Conversion Process

| Ex. No. | T Bed | T exo | P gauge | GHSV | CO/$H_2$/$N_2$ | Contraction | Time | Total Liquid Organic Product (gms/hr) |
|---------|-------|-------|---------|------|----------------|-------------|------|----------------------------------------|
| Fischer-Tropsch alone | | | | | | | | |
| 1 | 180 | 185 | 1.04 | 175 | 58/59/0 | 7% | – | – |
| 2 | 202 | 209 | 1.04 | 175 | 57/59/0 | 59% | 2 hr. | 0.20 |
| 3 | 196 | 202 | 1.04 | 168 | 54/58/0 | 45% | 2 hr. | 0.14 |
| 4 | 195 | 202 | 1.04 | 250 | 53/114/0 | 40% | 2 hr. | 0.43 |
| 5 | 256 | 268 | 1.04 | 210 | 65/75/0 | 70% | 3 hr. | 0.66 |
| 6 | 257 | 276 | 1.04 | 300 | 65/136/0 | 78% | 2 hr. | 1.00 |
| 7 | 224 | 233 | 1.04 | 1200 | 98/98/0 | ~25% | 2 hr. 2 min. | 0.25 |
| 8 | 224 | 230 | 1.04 | 1200 | 101/101/0 | 20% | 20 hr. | 0.16 |
| 9 | 237 | 247 | 1.04 | 639 | 107/106 | 7% | 18 hr. | 0.17 |
| 10 | 237 | 247 | 1.04 | 900 | 107/212 | 16% | 4 hr. 32 min. | 0.19 |
| 11 | 250 | 262 | 1.04 | 250 | 50/50/0 | 31% | 18 hr. 10 min. | 0.33 |
| 12 | 250 | 261 | 1.04 | 375 | 107/52/0 | 22% | 21 hr. 38 min. | 0.05 |
| Catalyst B | | | | | | | | |
| 13 | – | – | 0.7 | 900 | 0/300/0 | – | – | – |
| 14 | 211 | 219 | 1.04 | 300 | 50/50/0 | 51.1% | 16 hr. 9 min. | 0.37 |
| 15 | 204 | 218 | 1.04 | 450 | 50/100/0 | 67.7% | 23 hr. 25 min. | 0.35 |

0154063

TABLE III (Continued)

Synthesis Gas Conversion Process

| Ex. No. | T Bed | T exo | P gauge | GHSV | CO/H$_2$/N$_2$ | Contraction | Time | Total Liquid Organic Product (gms/hr) |
|---|---|---|---|---|---|---|---|---|
| Catalyst C | | | | | | | | |
| 16 | - | - | 0.7 | 900 | /300/ | - | 21 hr. | - |
| 17 | 223 | 231 | 1.04 | 300 | 50/50/0 | 24.8% | 3 hr. 23 min. | 0 |
| 18 | 237 | 248 | 1.04 | 300 | 50/50/0 | 29.2% | 15 hr. 54 min. | 0.22 |
| 19 | 237 | 247 | 1.04 | 450 | 50/100/0 | 26.9% | 6 hr. 54 min. | 0.16 |
| Catalyst D | | | | | | | | |
| 20 | - | - | 0.7 | 900 | 0/300/0 | - | 16 hr. | - |
| 21 | 230 | 240 | 1.04 | 300 | 50/50/0 | 55.6% | 17 hr. 28 min. | 0.50 |
| 22 | 215 | 224 | 1.04 | 450 | 50/100/0 | 45.4% | 3 hr. 6 min. | 0.34 |
| Catalyst E | | | | | | | | |
| 23 | - | - | 0 | 300 | 50/50/900 | - | 21 hr. 30 min. | - |
| 24 | 242 | 248 | 1.04 | 300 | 50/50/0 | 15.5% | 4 hr. 43 min. | 0 |
| 25 | 240 | 248 | 1.04 | 300 | 50/50/0 | 11.5% | 15 hr. 31 min. | 0.02 |
| 26 | 270 | 282 | 1.04 | 300 | 50/50/0 | 37.4% | 25 hr. 39 min. | 0.07 |
| 27 | 268 | 283 | 4.2 | 300 | 50/100/0 | 25.0% | 16 hr. | 0.07 |
| 28 | 270 | 285 | 3.45 | 1130 | 170/200/0 | 18.1% | 5 hr. 12 min. | 0.04 |
| Catalyst F | | | | | | | | |
| 29 | 240 | - | 0 | 300 | 50/50/900 | - | - | - |
| 30 | 251 | 261 | 1.04 | 300 | 50/50/0 | 29.4% | 3 hr. 37 min. | 0.17 |
| 31 | 260 | 272 | 1.04 | 300 | 50/50/0 | 45.7% | 15 hr. 37 min. | 0.35 |

TABLE III (Continued)

Synthesis Gas Conversion Process

| Ex. No. | T Bed | T exo | P gauge | GHSV | CO/H$_2$/N$_2$ | Contraction | Time | Total Liquid Organic Product (gms/hr) |
|---------|-------|-------|---------|------|----------------|-------------|------|----------------------------------------|
| Catalyst G | | | | | | | | |
| 32 | – | – | 0 | 3000 | 50/50/900 | – | 24 hr. | – |
| 33 | 241 | 259 | 1.04 | 300 | 50/50/0 | 57.3% | 3 hr. 51 min. | 0.30 |
| 34 | 241 | 259 | 1.04 | 300 | 50/50/0 | 65.2% | 16 hr. 15 min. | 0.36 |
| Catalyst A | | | | | | | | |
| 35 | – | – | 0 | 3000 | 50/50/900 | – | >10 hr. | – |
| 36 | 270 | 278 | 1.04 | 300 | 50/50/0 | 10.7% | 19 hr. 19 min. | 0.06 |

-15-

0154063

TABLE IV

Liquid Products From Synthesis Gas Conversion

| Ex. No. | SIMDIS °C 26% | SIMDIS °C 50% | SIMDIS °C 96% | VOL. AVG B.P. °C | % < 204° | $CH_2/CH_3$ | Vinyl Olefin | Internal Olefin | Vinylidene Olefin | Trisub Olefin | Total | Alc. | Acid | Ar. |
|---|---|---|---|---|---|---|---|---|---|---|---|---|---|---|
| Fischer-Tropsch Catalyst Alone | | | | | | | | | | | | | | |
| 1 | – | – | – | – | – | – | 14.4 | 4.34 | – | – | 18.74 | 1.58 | 2.18 | – |
| 7 | 189 | 252 | 404 | 254 | 33 | 6.5 | 18.7 | 6.2 | – | – | 24.9 | 10.1 | – | – |
| 9 | 170 | 236 | 416 | 251 | 37 | 5.7 | 19.4 | 2.8 | 1.2 | 2.4 | 25.8 | 19.4 | 11.6 | 1.4 |
| 10 | – | – | – | – | – | 5.3 | 15.3 | 4.0 | 1.0 | 1.0 | 21.3 | 29.4 | 2.2 | 0.5 |
| 11 | 121 | 192 | 432 | 213 | 54 | 3.8 | 8.0 | 8.3 | 0.9 | 1.0 | 18.2 | 8.8 | 4.7 | 0.7 |
| 12 | – | – | – | – | – | 4.1 | 4.4 | 8.1 | 0.9 | 1.0 | 14.4 | 15.1 | 12.2 | 0.9 |
| Catalyst B | | | | | | | | | | | | | | |
| 14 | 121 | 173 | 348 | 182 | 62 | 3.1 | 7.1 | 34.7 | .7 | 4.5 | 47 | 1.9 | 0 | 0 |
| 15 | 123 | 175 | 331 | 181 | 61 | 3.7 | 2.3 | 6.8 | – | 0.8 | 9.9 | 0 | 0 | 0 |
| Catalyst C | | | | | | | | | | | | | | |
| 18 | 129 | 192 | 370 | 200 | 56 | 4.9 | 6.7 | 13.2 | 0.2 | 1.2 | 21.3 | 23.2 | 0 | 0 |
| 19 | – | – | – | – | – | 4.4 | 5.8 | 11.7 | 0.6 | 1.5 | 19.6 | 21.0 | 0 | 0 |
| Catalyst D | | | | | | | | | | | | | | |
| 21 | 92 | 146 | 365 | 160 | 70 | 2.6 | 6.7 | 28.7 | 2.8 | 19.2 | 57.4 | 0 | 0 | 1.0 |
| 22 | – | – | – | – | – | 3.1 | 6.4 | 19.9 | 1.2 | 9.0 | 36.5 | 0 | 0 | 2.4 |
| Catalyst F | | | | | | | | | | | | | | |
| 30 | – | – | – | – | – | 2.8 | 9.0 | 6.8 | 0.8 | 2.0 | 18.6 | 25.3 | 0 | 4.0 |
| 31 | – | – | – | – | – | 3.4 | 7.8 | 9.7 | 1.2 | 3.3 | 22.0 | 7.8 | 0 | 2.4 |

## TABLE IV (Continued)

### Liquid Products From Synthesis Gas Conversion

| Ex. No. | SIMDIS °C 26% | 50% | 96% | VOL. AVG B.P. °C | % < 204° | $CH_2/CH_3$ | Vinyl Olefin | Internal Olefin | Vinylidene Olefin | Trisub Olefin | Total | Alc. | Acid | Ar. |
|---------|-----|-----|-----|------|------|------|------|------|------|------|------|------|------|------|
| Catalyst G | | | | | | | | | | | | | | |
| 34 | 122 | 172 | 347 | 182 | 63 | 3.3 | 9.2 | 18.6 | 1.0 | 4.1 | 32.9 | 2.5 | 0 | 2.4 |
| Catalyst A | | | | | | | | | | | | | | |
| 36 | 129 | 163 | 370 | 182 | 70 | 2.1 | 0 | 3.6 | 0 | 2.8 | 6.4 | 0 | 0 | 22.6 |

0154063

TABLE V

Conversion of CO and Selectivity of Catalysts A through G

| Ex. No. | 14 - Catalyst B | | | 15 - Catalyst B | | | 18 - Catalyst C | | | 19 - Catalyst C | | |
|---|---|---|---|---|---|---|---|---|---|---|---|---|
| | SCCM | %PPC | %SEL | SCCM | %PPC | %SEL | SCCM | %PPC | %SEL | SCCM | %PPC | %SEL |
| Products | | | | | | | | | | | | |
| CO (conv) | 21.30 | – | – | 34.55 | – | – | 18.99 | – | – | 22.04 | – | – |
| CO | 28.70 | – | – | 15.45 | – | – | 31.01 | – | – | 27.96 | – | – |
| $CO_2$ | 0.24 | 0.48 | 1.13 | 0.27 | 0.54 | 0.78 | 4.53 | 9.06 | 23.85 | 5.59 | 11.18 | 25.36 |
| $CH_4$ | 0.98 | 1.96 | 4.60 | 3.41 | 6.82 | 9.87 | 1.84 | 3.68 | 9.69 | 4,06 | 8.12 | 18.42 |
| $C_2H_6$ | 0.41 | 0.82 | 1.92 | 0.62 | 1.24 | 1.79 | 0.54 | 1.08 | 2.84 | 1.16 | 2.32 | 5.26 |
| $C_2H_4^=$ | – | – | – | – | – | – | – | – | – | – | – | – |
| $C_3H_8$ | 0.37 | 0.74 | 1.74 | 1.01 | 2.02 | 2.92 | 0.25 | 0.50 | 1.32 | 0.66 | 1.32 | 2.99 |
| $C_3H_6^=$ | 0.94 | 1.88 | 4.41 | 0.33 | 0.66 | 0.95 | 0.96 | 1.92 | 5.06 | 1.35 | 2.70 | 6.12 |
| $i-C_4H_{10}$ | – | – | – | 0.02 | 0.04 | 0.06 | – | – | – | – | – | – |
| $nC_4H_{10}$ | 0.35 | 0.70 | 1.64 | 0.94 | 1.88 | 2.72 | 0.23 | 0.46 | 1.21 | 0.53 | 1.06 | 2.40 |
| $1-C_4H_8^=$ | 0.33 | 0.66 | 1.55 | 0.16 | 0.32 | 0.46 | 0.54 | 1.08 | 2.84 | 0.53 | 1.06 | 2.40 |
| $i-C_4H_8^=$ | 0.02 | 0.04 | 0.09 | 0.02 | 0.04 | 0.06 | – | – | – | – | – | – |
| $t-C_4H_8^=$ | 0.39 | 0.78 | 1.83 | 0.14 | 0.28 | 0.41 | – | – | – | 0.22 | 0.44 | 1.00 |
| $c-C_4H_8^=$ | 0.27 | 0.54 | 1.27 | 0.10 | 0.20 | 0.29 | 0.06 | 0.12 | 0.32 | 0.22 | 0.44 | 1.00 |
| $i-C_5H_{12}$ | – | – | – | – | – | – | – | – | – | – | – | – |
| $nC_5H_{12}$ | – | – | – | 0.05 | 0.10 | 0.14 | – | – | – | – | – | – |
| Liq. gas equiv. | – | 19.84 | 46.57 | – | 18.82 | 27.23 | – | 11.90 | 31.33 | – | 8.64 | 19.60 |

0154063

TABLE V (Continued)

Conversion of CO and Selectivity of Catalysts A through G

| Ex. No. | 21 – Catalyst D | | | 22 – Catalyst D | | | 25 – Catalyst E | | | 26 – Catalyst E | | |
|---|---|---|---|---|---|---|---|---|---|---|---|---|
| | SCCM | %PPC | %SEL | SCCM | %PPC | %SEL | SCCM | %PPC | %SEL | SCCM | %PPC | %SEL |
| **Products** | | | | | | | | | | | | |
| CO (conv) | 20.8 | – | – | 21.4 | – | – | 8.92 | – | – | 29.53 | – | – |
| CO | 29.2 | 58.4 | – | 28.6 | 57.2 | – | 41.08 | – | – | 20.47 | – | – |
| $CO_2$ | .85 | 1.7 | 4.1 | .33 | .67 | 1.6 | 3.58 | 7.16 | 40.13 | 14.15 | 28.30 | 42.92 |
| $CH_4$ | 2.0 | 3.9 | 9.5 | 3.2 | 6.5 | 15.2 | 1.79 | 3.58 | 20.07 | 4.76 | 9.52 | 16.12 |
| $C_2H_6$ | .95 | 1.9 | 4.5 | 0.95 | 1.9 | 4.5 | 1.47 | 2.94 | 16.48 | 3.13 | 6.26 | 10.60 |
| $C_2H_4^=$ | – | – | – | – | – | – | 0.23 | 0.46 | 2.58 | – | – | – |
| $C_3H_8$ | 0.7 | 1.4 | 3.4 | 0.9 | 1.8 | 4.1 | 0.62 | 1.24 | 6.95 | 1.24 | 2.48 | 4.20 |
| $C_3H_6^=$ | 1.1 | 2.1 | 5.0 | 1.1 | 2.1 | 5.0 | 0.72 | 1.44 | 8.07 | 0.28 | 0.56 | 0.95 |
| $i\text{-}C_4H_{10}$ | – | – | – | – | – | – | 0.04 | 0.08 | 0.45 | 0.20 | 0.40 | 0.68 |
| $nC_4H_{10}$ | .41 | .83 | 2.0 | 0.7 | 1.4 | 3.3 | 0.29 | 0.58 | 3.25 | 0.58 | 1.16 | 1.96 |
| $1\text{-}C_4H_8^=$ | .22 | .45 | 1.1 | 0.30 | .60 | 1.4 | 0.11 | 0.22 | 1.23 | 0.65 | 1.30 | 2.20 |
| $i\text{-}C_4H_8^=$ | .10 | .19 | .46 | 0.12 | .24 | .55 | 0.14 | 0.28 | 1.57 | 0.40 | 0.80 | 1.36 |
| $t\text{-}C_4H_8^=$ | .65 | 1.3 | 3.1 | 0.65 | 1.3 | 3.1 | 0.39 | 0.78 | 4.37 | 0.23 | 0.46 | 0.78 |
| $c\text{-}C_4H_8^=$ | .38 | .75 | 1.8 | 0.40 | .79 | 1.8 | 0.21 | 0.42 | 2.35 | 0.13 | 0.26 | 0.44 |
| $i\text{-}C_5H_{12}$ | – | – | – | – | – | – | – | – | – | 0.25 | 0.50 | 0.85 |
| $nC_5H_{12}$ | – | – | – | – | – | – | – | – | – | – | – | – |
| Liq. gas equiv. | – | 26.4 | 63.6 | – | 18.2 | 42.6 | – | 1.12 | 6.28 | – | 3.52 | 5.96 |

0154063

TABLE V (Continued)

Conversion of CO and Selectivity of Catalysts A through G

| Ex. No. | 27 - Catalyst E | | | 30 - Catalyst F | | | 31 - Catalyst F | | |
|---|---|---|---|---|---|---|---|---|---|
| | SCCM | %PPC | %SEL | SCCM | %PPC | %SEL | SCCM | %PPC | %SEL |
| Products | | | | | | | | | |
| CO (conv) | 18.27 | – | – | 36.44 | – | – | 45.49 | – | – |
| CO | 31.73 | – | – | 13.56 | – | – | 4.51 | – | – |
| $CO_2$ | 3.71 | 7.42 | 20.31 | 14.97 | 29.94 | 41.08 | 24.05 | 8.10 | 52.80 |
| $CH_4$ | 5.63 | 11.26 | 30.82 | 2.12 | 4.14 | 5.82 | 3.20 | 6.40 | 7.04 |
| $C_2H_6$ | 3.38 | 6.76 | 18.50 | 1.55 | 3.10 | 4.25 | 2.72 | 5.44 | 5.98 |
| $C_2H_4^=$ | – | – | – | 0.12 | 0.24 | 0.33 | – | – | – |
| $C_3H_8$ | 2.00 | 4.00 | 10.95 | 0.59 | 1.18 | 1.62 | 1.25 | 2.50 | 2.75 |
| $C_3H_6^=$ | 0.10 | 0.20 | 0.55 | 1.65 | 3.30 | 4.53 | 2.28 | 4.56 | 5.01 |
| $i-C_4H_{10}$ | 0.14 | 0.28 | 0.77 | 0.03 | 0.06 | 0.08 | 0.04 | 0.08 | 0.09 |
| $nC_4H_{10}$ | 0.81 | 1.62 | 4.43 | 0.54 | 1.08 | 1.48 | 1.09 | 2.18 | 2.40 |
| $1-C_4H_8^=$ | – | – | – | 0.90 | 1.80 | 2.47 | 1.02 | 2.04 | 2.24 |
| $i-C_4H_8^=$ | – | – | – | 0.14 | 0.28 | 0.38 | 0.20 | 0.40 | 0.44 |
| $t-C_4H_8^=$ | – | – | – | – | – | – | 0.20 | 0.40 | 0.44 |
| $c-C_4H_8^=$ | – | – | – | – | – | – | 0.24 | 0.48 | 0.53 |
| $i-C_5H_{12}$ | 0.28 | 0.56 | 1.53 | – | – | – | – | – | – |
| $nC_5H_{12}$ | – | – | – | – | – | – | 0.46 | 0.92 | 1.01 |
| Liq. gas equiv. | – | 3.46 | 9.41 | – | 9.12 | 12.51 | – | 18.66 | 20.51 |

Table V (Continued)

Conversion of CO and Selectivity of Catalysts A through G

| Ex. No. | 34 – Catalyst G | | | 36 – Catalyst A | | |
|---|---|---|---|---|---|---|
| | SCCM | %PPC | %SEL | SCCM | %PPC | %SEL |
| Products | | | | | | |
| CO (conv) | 45.82 | – | – | 10.5 | – | – |
| CO | 4.18 | – | – | 38.5 | 77.0 | – |
| $CO_2$ | 15.87 | 31.74 | 34.63 | 5.4 | 10.7 | 46.5 |
| $CH_4$ | 1.77 | 3.48 | 3.86 | 2.6 | 5.1 | 22.1 |
| $C_2H_6$ | 1.46 | 2.92 | 3.19 | 1.6 | 3.3 | 14.4 |
| $C_2H_4^=$ | 0.19 | 0.38 | 0.41 | – | – | – |
| $C_3H_8$ | 0.58 | 1.16 | 1.27 | 0.8 | 1.6 | 6.9 |
| $C_3H_6^=$ | 1.90 | 3.96 | 4.32 | .06 | .13 | .6 |
| $i-C_4H_{10}$ | 0.03 | 0.06 | 0.06 | .30 | .60 | 2.6 |
| $nC_4H_{10}$ | 0.46 | 0.92 | 1.00 | .46 | .93 | 4.1 |
| $1-C_4H_8^=$ | 0.88 | 1.76 | 1.92 | – | – | – |
| $i-C_4H_8^=$ | 0.14 | 0.28 | 0.30 | .09 | .18 | .80 |
| $t-C_4H_8^=$ | 0.21 | 0.42 | 0.46 | – | – | – |
| $c-C_4H_8^=$ | 0.26 | 0.52 | 0.56 | – | – | – |
| $i-C_5H_{12}$ | – | – | – | .24 | .48 | 2.1 |
| $nC_5H_{12}$ | 0.10 | 0.20 | 0.22 | – | – | – |
| Liq. gas equiv. | – | 19.42 | 21.19 | – | 3.0 | 13.2 |

With reference to the tables, it can be seen first from Table III, that the Fischer-Tropsch catalysts produced the greatest amount of organic materials and that many of the other examples reported organic product yields higher than Catalyst A, Example No. 40.

In Table IV, it is seen that Catalyst A produced the lowest amount of olefins and no oxygenates while Catalysts B-G as well as the Fischer-Tropsch catalysts were particularly high in olefins. Catalysts C, D, F and G comprised unmodified silicalite and a Fischer-Tropsch component; for F and G, the same Fischer-Tropsch component was employed and Catalyst G employed the same silicalite selected for Catalyst A. Olefins that were produced by Catalyst A were internal and trisubstituted which are more useful than the others. More significantly, the highest yield of aromatics was obtained with Catalyst A.

It is also to be noted from Table IV that the best (lowest) $CH_2/CH_3$ ratio was also obtained. As stated hereinabove, the methylene to methyl ratio can be utilized to determine the chain length or extent of branching. For instance, comparing the ratio of a straight chain $C_{16}$ paraffin (7/1) to that for a $C_8$ paraffin (3.0/1) it appears that the longer chain has the higher number. However, when a $C_8$ paraffin is branched, such as iso-octane the ratio becomes 1/5 or 0.20, from which it is evident that branching lowers the ratio considerably.

By comparing the $CH_2/CH_3$ number with boiling point fraction data, i.e. SIMDIS (simulated distillation), volume average boiling point and percent boiling under 204° C, the usefulness of the product as a gasoline former or as gasoline can also be determined. Inasmuch as greater chain length increases the $CH_2/CH_3$ ratio while branching decreases it and longer chain compounds have higher boiling points, a ratio of about 2.5 coupled with a high percent of materials boiling under 204° C, is indicative of a good gasoline former while a value of 2.0 is excellent.

Based upon the satisfactory yields of liquid hydrocarbons, particularly the aromatics, the minimal amounts of olefins and lack of oxygenates that have been obtained when the catalyst of the present invention has been employed in the process set forth herein, it should be apparent that the objects of the invention have been met. It is to be understood that the catalyst of the present invention can be modified with other metals from Groups VIB or VIIB than chromium. Similarly, other Fischer-Tropsch catalysts can be employed.

It should be apparent to those skilled in the art that the process of the subject invention is operable with catalysts having fairly broad ratios of modifier to silica molecular sieve and of silica molecular sieve to Fischer-Tropsch catalyst and that the process is operable when other temperatures, pressures and times are employed. Similarly, parameters for the preparation of the catalyst can be varied from those which have been exemplified. It is to be understood that these variables fall within the scope of the claimed invention and that the subject invention is not to be limited by the examples set forth herein. These have been provided merely to demonstrate operability and, therefore, the selection of specific catalyst components and process conditions can be determined without departing from the spirit of the invention herein disclosed and described. Moreover, the scope of the invention shall include all modifications and variations that may fall within the scope of the attached claims.

CLAIMS:

1.    A catalyst composition comprising:

a silica molecular sieve modified by a metal which forms acidic oxides selected from the group consisting of Group VIB and VIIB elements; and

a Fischer-Tropsch catalyst wherein the ratio of silica molecular sieve to Fischer-Tropsch component is from 0.1 to 10:1.

2.    A catalyst as claimed in claim 1 characterised in that the amount of the metal in the silica molecular sieve comprises from 0.01 to 20 percent by weight.

3.    A catalyst as claimed in claim 1 or claim 2 characterised in that the elements are selected from Group VIB.

4.    A catalyst as claimed in claim 3 characterised in that the silica molecular sieve is modified with chromium in an amount of approximately three percent by weight and the modified silica molecular sieve is combined with an equal amount by weight of the Fischer-Tropsch catalyst.

5.    A catalyst as claimed in claim 4 characterised in that the Fischer-Tropsch catalyst comprises 75Fe(II): 25Fe(III):20Cu(II).

6.    A catalyst as claimed in claim 5 characterised in that it further comprises at least one percent by weight of a lubricant.

7.    A catalyst as claimed in claim 6 characterised in that the lubricant is graphite.

8.    A catalyst as claimed in any of claims 1 to 7 characterised in that the silica molecular sieve comprises a substantially aluminum free zeolite having a silica to aluminum molar ratio of more than 500:1.

9.    A catalyst as claimed in claim 8 characterised in that the silica molecular sieve is silicalite.

10.   A process for producing gasoline quality liquid hydrocarbons comprising the steps of:

contacting synthesis gas having a ratio of hydrogen to carbon oxides of from 0.1 to 10.0:1 over a combination catalyst at a reaction temperature of from 100°C to 500°C and at a pressure of from 0.1 MPa to 20 MPa and at a gas hourly space velocity of from 10 to 100,000, characterised in that the combination catalyst is a composition as claimed in any of claims 1 to 9.

11. A process as claimed in claim 10 characterised in that the synthesis gas composition is 1:1 $H_2$/CO.

12. A process as claimed in claim 11 characterised in that the temperature is 250°C, the pressure is 1.04 MPa and the GHSV is 300.

13. A process as claimed in any of claims 10 to 12 characterised in that it includes the further step of reducing the combination catalyst with $H_2$ and/or CO at a temperature of from 100°C to 600°C, at $H_2$ and/or CO partial pressures ranging from 0.001 MPa to 5.07 MPa and for a period of time of from one to 100 hours prior to said step of contacting.

14. A method for the preparation of a catalyst composition as claimed in any of claims 1 to 9 characterised in that one modifies a silica molecular sieve with a metal which forms acidic oxides selected from the group consisting of Group VIB and VIIB elements; and

combines the modified silica molecular sieve with a Fischer-Tropsch catalyst in a ratio of from 0.1 to 10:1.

15. A method as claimed in claim 14 characterised in that it includes the further step of combining at least one percent by weight of a lubricant with the modified silica molecular sieve and the Fischer-Tropsch catalyst, the lubricant preferably being graphite.

16. A method as claimed in claim 14 or claim 15 characterised in that it further comprises the step of precalcining the silica molecular sieve at temperatures ranging from 100° to 800°C for at least one hour prior to the step of modification.

17. A method as claimed in any of claims 14 to 16 characterised in that the step of modifying includes the steps of impregnating the silica molecular sieve with a solution of the metal; evaporating the solvent from the solution; and calcining the modified silica molecular sieve at temperatures within the range of from 300° to 800°C for one to 24 hours.

18. A method as claimed in any of claims 14 to 17 characterised in that it includes the further step of reducing said combination catalyst with $H_2$ and/or CO at a temperature of from 100°C to 600°C, at $H_2$ and/or CO partial pressures ranging from 0.001 MPa to 5.07 MPa and for a period of time of from one to 100 hours prior to employing said catalyst for the conversion of synthesis gas.

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-2 099 716 (RESEARCH ASSOCIATION)<br><br>* page 2, lines 58-62; page 2, lines 43-65; page 3, lines 1-9, 16-24 * | 1-3,10 -12,14 -17 | C 07 C 1/04<br>B 01 J 29/06 |
| Y | | 1,4-10 ,15 | |
| D,Y | FR-A-2 268 771 (MOBIL OIL)<br><br>* page 2, lines 23-33; page 5, lines 11-23, 33-34; page 9, lines 16-20; page 11, lines 24-30; page 13, lines 33-34 * | 1,4,6, 7,10, 15 | |
| A | | 5,13, 18 | TECHNICAL FIELDS SEARCHED (Int. Cl.4)<br><br>C 07 C<br>B 01 J |
| Y | EP-A-0 099 715 (BRITISH PETROLEUM)<br>* abstract; page 4, lines 4-7 * | 8,9 | |
| A | | 13-18 | |
| A | FR-A-2 320 923 (AIR PRODUCTS)<br>* page 3, lines 27-29 * | 5 | |
| | ---         -/- | | |

The present search report has been drawn up for all claims

| Place of search | Date of completion of the search | Examiner |
|---|---|---|
| THE HAGUE | 02-11-1984 | SALA P.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

| | DOCUMENTS CONSIDERED TO BE RELEVANT | | Page 2 |
|---|---|---|---|

| Category | Citation of document with indication. where appropriate, of relevant passages | Relevant to claim | CLASSIFICATION OF THE APPLICATION (Int. Cl.4) |
|---|---|---|---|
| Y | FR-A-1 065 567 (RUHRCHEMIE) <br> * page 1, right-hand column, lines 33-40; page 2, right-hand column, lines 56-57; page 3, left-hand column, lines 1-7, 20-34 * <br><br> ----- | 5 | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 02-11-1984 | SALA P.C. |